# EUROPEAN PATENT SPECIFICATION

(11) **EP 0 451 452 B1**
(45) Date of publication and mention of the grant of the patent: **13.12.1995**
(21) Application number: 91101675.6
(22) Date of filing: 07.02.1991
(51) Int. Cl.: C12N 15/70, C12N 15/55, C12N 9/20

(54) **A recombinant plasmid for lipase expression and a process for producing a lipase**
Rekombinantes Plasmid für die Expression von Lipase und Verfahren zur Herstellung von Lipase
Plasmide recombinante pour l'expression de lipase et procédé de production de lipase

(30) Priority: 07.03.1990 JP 55859/90
(43) Date of publication of application: 16.10.1991
(73) Proprietor: CHISSO CORPORATION, Osaka (JP)
(72) Inventor: Aoyama, Shigeyuki, Ichiharashi, Chibaken (JP); Zenno, Shuhei, Yokohamashi, Kanagawaken (JP); Inouye, Satoshi, Chiyoda-ku, Tokyo (JP); Yoshida, Naoyuki, Ichiharashi, Chibaken (JP)
(74) Representative: Kraus, Walter, Dr.

(56) References cited:
- EP-A- 0 152 613
- EP-A- 0 237 338
- EP-A- 0 318 775
- GENE, vol. 25, nos. 2,3, part II, November 1983, pages 167-178, Amsterdam, NL; E. AMANN et al.: "Vectors bearing a hybrid trp-lac promoter useful for regulated expression of cloned genes in Escherichia coli"
- BIOCHEMICAL AND BIOPHYSICAL RESEARCH COMMUNICATIONS, vol. 141, no. 1, 26 November 1986, pages 185-190, New York, US; W. KUGIMIYA et al.: "Molecular cloning and nucleotide sequence of the lipase gene from pseudomonas fragi"
- PROCEEDINGS OF THE NATIONAL ACADEMY OF SCIENCES OF THE UNITED STATES OF AMERICA, vol. 80, no. 1, January 1983, pages 21-25, Washington DC, US; H.A. DE BOER et al.: "The tac promoter: A functional hybrid derived from the trp and lac promoters"

## Description

### BACKGROUND OF THE INVENTION

### 1. Field of the Invention

This invention relates to a recombinant plasmid capable of effecting expression of a lipase originated from Pseudomonas fragi IFO 12049 strain (hereinafter abbreviated merely to lipase) inside Eschericia coli in a good efficiency, an Escherichia coli transformed by the above recombinant plasmid and a process for producing the above lipase.

More particularly, the present invention relates to a recombinant plasmid capable of effecting a lipase expression inside Escherichia coli in a good efficiency, characterized in that, when a lipase gene is inserted into an expression vector capable of effecting expression of a foreign gene inside Escherichia coli according to gene manipulation, to construct a new recombinant plasmid, then a fused promoter (tac promoter) of a promoter of Escherichia coli tryptphan operon with Escherichia coli lactose operon, is used as a vector of multicopy type which is replicative inside Escherichia coli; an Escherichia coli strain transformed by the above plasmid; and further a process for producing a lipase using the Escherichia coli strain.

### 2. Description of the Related Art

Lipases have been used as a lipid-hydrolyzing enzyme, for oils- and fats-processing, medicine for clinical diagonosis, detergents, digestants, etc. Further, in recent years, they are important enzymes which catalyze ester hydrolysis, ester synthesis or ester conversion in the production of chemical products, particularly optically active compounds.

Further, Pseudomonas genus bacterium has been known to produce lipases useful as a catalyst for ester hydrolysis, ester synthesis or ester conversion (Japanese patent application laid-open No. Sho 62-166,898/1987).

The specific feature of the lipases produced by the Pseudomonas genus bacterium is interesting in the aspect of commercial utilization, but its production is effected by expression of usually one gene contained in the chromosome DNA of the lipase-producing bacterium; hence it has been difficult to improve the production of the lipase with a leap.

In recent years, due to development of recombinant DNA technique, it has become possible to derive genes into those having a higher activity through its isolation or to transform nucleotide sequence; thus it has been investigated or conducted to create commercially interested proteins.

Particularly when production of a useful protein according to recombinant DNA technique is investigated, it is very effective to use Escherichia coli as host since various factors relative to the production of the substance have been comprehended and the development and improvement of the expression vector have been carried out.

The present inventors have previously isolated a lipase gene from Pseudomonas fragi IFO 12049 strain, determined its primary structure and succeeded in confirming the expression of a lipase within Escherichia coli as a host (Japanese patent application No. Sho 63-187,684). However, the quantity thereof expressed was so small that it has been urgent to choose and develop a recombinant plasmid-host system capable of effecting expression of a lipase within Escherichia coli in a good efficiency.

From such a viewpoint, in order to develop a suitable expression plasmid for effecting expression of a lipase within Escherichia coli in good efficiency, the present inventors have noted and made research in the number of copies of the expression within Escherichia coli and the promoter. As a result, when a replication origin of a multicopy pUC type plasmid is used as the replicator of the expression vector and tac promoter is also used as the promoter, then it has been found that it is possible to realize expression of the lipase within Escherichia coli in good efficiency, and the present invention has been completed based upon the above finding.

### SUMMARY OF THE INVENTION

As apparent from the foregoing, the object of the present invention is to provide a recombinant plasmid capable of effecting expression of a lipase within Escherichia coli in a good efficiency in the expression of a lipase within Escherichia coli according to gene manipulation DNA technique, Escherichia coli strains transformed by the plasmid and suitable for producing a lipase in a good efficiency and a process for producing a lipase in the best efficiency in accordance with characteristics specific of such Escherichia coli strains.

The present invention consists in the following constitutions:
(1) A recombinant plasmid for lipase expression comprising
   (a) a vector of a multicopy type replicative inside Escherichia coli,
   (b) a fused promoter (tac promoter) of a promoter of Escherichia coli tryptphan operon with a promoter of Escherichia coli lactose operon, and
   (c) the following DNA sequence coding a lipase originated from Pseudomonas fragi IFO 12049 strain under the control of said fused promoter: 
10 20 30 40 50 60
ATGGACGATT CGGTAAATAC ACGCTATCCG ATCTTATTGG TACACGGCCT TTTTGGATTC

70 80 90 100 110 120
GACCGGATAG GCTCGCATCA CTACTTTCAT GGTATCAAGC AAGCACTCAA TGAGTGCGGT

130 140 150 160 170 180
GCCAGCGTCT TCGTTCCAAT CATTTCAGCC GCCAACGACA ATGAAGCCCG AGGCGATCAA

190 200 210 220 230 240
CTGCTCAAGC AGATCCACAA CCTGCGCAGG CAGGTCGGTG CACAGCGTGT CAACCTGATC

250 260 270 280 290 300
GGCCACAGCC AGGGCGCCCT TACCGCGCGT TATGTGGCGG CCATCGCCCC TGAACTGATC

310 320 330 340 350 360
GCCTCGGTGA CGTCAGTCAG TGGCCCCAAC CACGGCTCCG AGCTGGCCGA TCGCTTGCGC

370 380 390 400 410 420
CTGGCCTTTG TCCCGGGGAG GCTTGGCGAA ACGGTGGCTG CCGCCCTGAC CACCTCGTTC

430 440 450 460 470 480
AGCGCATTTT TATCCGCCCT CAGCGGCCAC CCGCGCCTGC CCCAAAATGC CTTGAATGCG

490 500 510 520 530 540
CTTAACGCCC TGACCACCGA TGGGGTTGCC GCCTTCAACC GCCAATATCC CCAGGGGCTG

550 560 570 580 590 600
CCCGACAGAT GGGGCGGCAT GGGCCCCGCA CAGGTGAATG CTGTGCACTA TTATTCCTGG

610 620 630 640 650 660
AGCGGCATCA TCAAAGGCTC GCGGCTGGCC GAGTCACTCA ACCTGCTCGA CCCCTTGCAC

670 680 690 700 710 720
AATGCCTTGC GTGTGTTTGA CAGCTTTTTT ACCCGCGAAA CCCGAGAAAA CGACGGCATG

730 740 750 760 770 780
GTTGGCCGCT TCAGCTCGCA TCTGGGCCAG GTCATCCGCT CCGACTACCC TCTGGACCAC

790 800 810 820 830 840
CTCGACACCA TCAACCATAT GGCAAGAGGA AGCGCAGGCG CATCAACCCG GTAG
(2) An Escherichia coli strain transformed by a recombinant plasmid as set forth in item (1).
(3) A process for producing a lipase, which comprises using an Escherichia coli strain as set forth in item (2).

### BRIEF DESCRIPTION OF THE DRAWINGS

Fig. 1 shows a flowsheet concretely illustrating the construction steps of plasmid pTC-1.

Fig. 2 shows a flowsheet concretely illustrating the construction steps of plasmid pTCE-lip and pTCS-lip.

Fig. 3 shows a DNA sequence between SD sequence and lipase-initiation codon ATG in plasmid pTCE-lip and plasmid pTCS-lip.

### DETAILED DESCRIPTION OF PREFERRED EMBODIMENTS

The constitutions and effectiveness of the present invention will be described below.

As the vector used for the recombinant plasmid of the present invention, it is possible to use a DNA fragment retained stably inside Escherichia coli as a host and having a plasmid replicator (replication origin) which is of multicopy type, replicative i.e. effectively functionable inside Escherichia coli. As the plasmid replicator, it is possible to use those of pUC type plasmid, etc.

Further, these plasmids are convenient to contain genes constituting a selective marker when a recombinant plasmid is introduced into a host bacterium, for example, various genes capable of effecting expression of resistance to Ampicillin or Kanamycin, etc.

The tac promoter used for the recombinant plasmid of the present invention may be constructed from trp promoter and lac promoter according to the method reported by E. Amann et al (Gene, 25, 167 (1983)).

Further, the tac promoter may be cut out from a suitable vector containing tac promoter as exemplified by vector-pKK 223-3 (made by Pharmacia Co., Ltd.). In addition, the tac promoter is preferred to contain SD (Shine-Dalgarno) sequence.

Further, in order that this tac promoter effectively functions for expression of lipase gene, the sequence between SD sequence of tac promoter and initiation codon ATG of lipase gene should also be taken into consideration. For example, a sequence as illustrated by the present inventors in Example mentioned later is preferred.

In the recombinant plasmid of the present invention, as a DNA sequence coding a lipase gene incorporated
downstream of tac promoter, a sequence already cloned from Pseudomonas fragi IFO 12049 strain by the present inventors and disclosed in Japanese patent application No. Sho 63-187,684 (which is a DNA sequence described in the above constitution (1)) may be utilized, and a DNA sequence coding a lipase gene which is functionally equivalent to the above gene may also be, of course, utilized.

In the recombinant plasmid of the present invention, as a terminator for mRNA incorporated so as to be positioned downstream of the DNA sequence coding a lipase gene, any of those which effectively function within Escherichia coli may be used, but use of a terminator exhibiting a high activity e.g. rrnB or trpA is preferred in the aspect of balancing it with a promoter having a high promoter activity such as tac promoter.

When the recombinant plasmid of the present invention having the above constitution is introduced into Escherichia coli as a host bacterium, it is possible to effect expression of a lipase having a higher efficiency within the Escherichia coli as a host.

As to the expression of lipase, if a lactose repressor-producing bacterium is used as a host bacterium, it is possible to induce the expression by means of lactose or isopropyl-β-D-thiogalactopyranoside (IPTG).

As examples of this bacterium, JM 109 [Yanisch-Perron, C., Vieira, J. and Messing, J, Gene, 33, 103 (1985)], D 1210 [Sadler, S.J., Tecklenburg, M. and Betz, J.L., Gene, 8, 279 (1980)], etc. may be utilized.

Further, if a bacterium producing no lactose repressor is used as a host bacterium, it is possible to effect expression of lipase even when an inducing substance such as IPTG is not used. As examples of this bacterium, JM 83 [Messing, J., Crea, R. and Seeberg, P.H., Nucl. Acid. Res., 2, 309 (1981)], etc. may be utilized.

Anyhow, culture conditions may be determined so as to be able to effect lipase expression in a good efficiency, according to the characteristics specific of Escherichia coli as a host.

A sample of Escherichia coli adjusted under culture conditions capable of effecting lipase expression in a good efficiency as described above, has its constituting components separated up to a polypeptide level, according to sodium dodecyl sulfate (SDS)-polyacrylamide gel electrophoresis.

The thus separated polypeptide is then dyed in a suitable manner and visualized. Confirmation of the lipase expression within Escherichia coli by the recombinant plasmid of the present invention is carried out by comparing the pattern of the SDS-polyacrylamide gel electrophoresis, using as a control, the same adjusted sample of Escherichia coli containing a plasmid having no lipase gene incorporated thereinto.

Further, evaluation of the lipase activity expressed within Escherichia coli may be carried out according to a method of determination by titrating a fatty acid liberated by a lipase function using emulsified olive oil as a substrate, or a method of using a commercially available lipase activity measurement kit, etc.

Thus, according to the present invention, it has become possible to express a lipase gene originated from a Pseudomonas bacterium within Escherichia coli in a better efficiency, and also it has become possible to provide the same lipase as a lipase originated from a Pseudomonas bacterium utilizable for commercial application uses, or its variant retaining substantially the same specific features.

The present invention will be described in more detail by way of Examples, but it should not be construed to be limited thereto.

### Example 1

### (Construction of plasmid pTCE-lip·pTCS-lip)

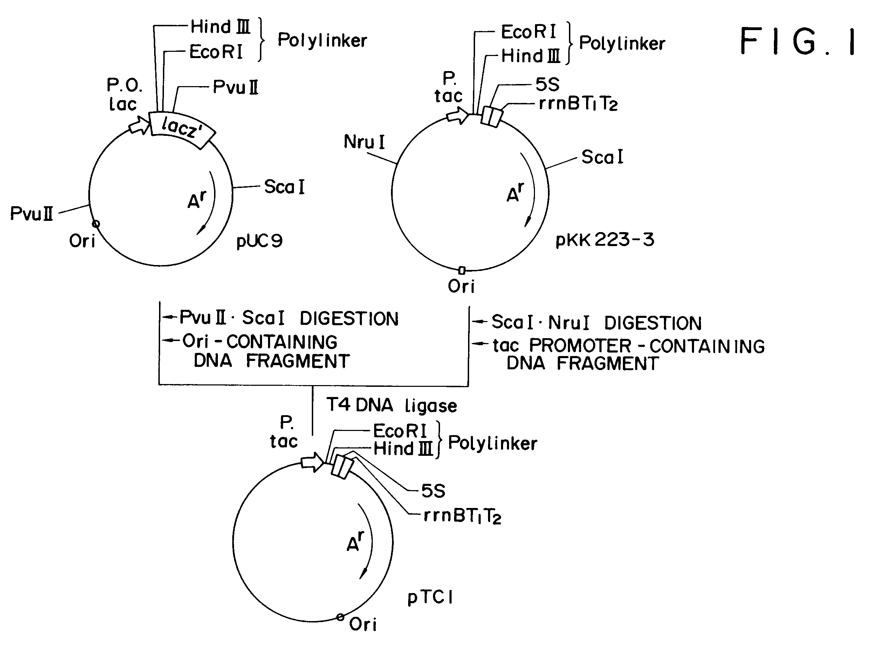

### (i) Construction of plasmid pTC1 according to the steps of Fig. 1:

Firstly, a replication origin (ori)-containing DNA fragment was separated and recovered from a mixture of DNA fragments obtained by digesting plasmid pUC9 [Vieira, J. and Messing, J., Gene 19, 259 (1982), Yanisch-Perron, C., Vieira, J. and Messing, J., Gene 33, 103 (1985)], with a restriction enzyme Pvu II·Sca I, according to a known electrophoresis method, while a tac promoter-containing DNA fragment was separated and recovered from a mixture of DNA fragments obtained by digesting plasmid pKK223-3 (made by Pharmacia Co., Ltd.) with a restriction enzyme ScaI·NruI, according to a known electrophoresis method.

Next, the thus obtained two DNA fragments were reacted in the presence of T4 DNA ligase, followed by introducing the resulting reaction product into Escherichia coli according to Hanahan's method [Hanahan, D., J. Mol. Biol., 166, 557 (1983)] and culturing the resulting product on an LB medium plate containing 50 »g/mℓ of Ampicillin ( the medium: 1% bactotripton·0.5% yeast extract·0.5% NaCℓ·1.5% agar, adjusted to a pH of 7.2). After the culture, plasmids were indivisually extracted from the Ampicillin-resistant colonies which appeared on the plate, followed by preparing a restriction map of the respective plasmids, identifying a colony having plasmid pTC1 of a construction as shown in Fig. 1 and isolating the plasmid pTC1 from the colony.

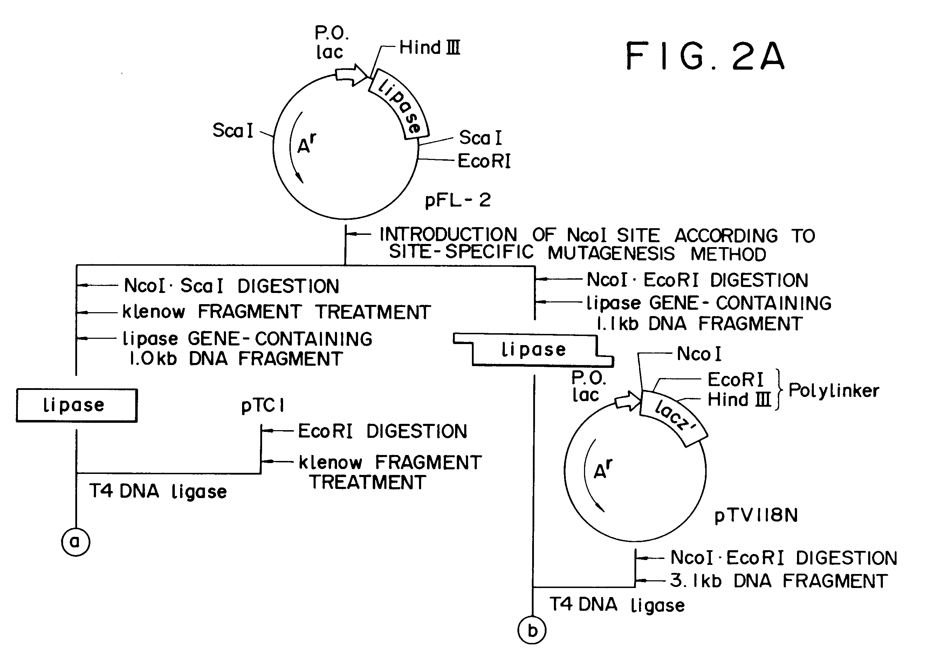

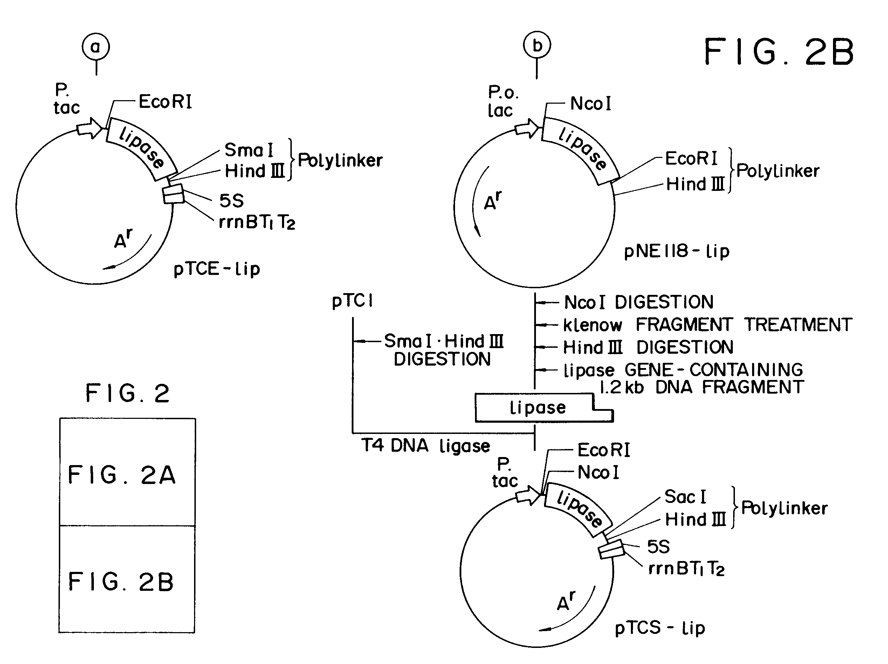

### (2) Construction of plasmids pTCE-lip and pTCS-lip illustrated in the steps of Fig. 2:

### (2-1) Construction of pTCE-lip

Firstly, the NcoI site of a restriction enzyme was introduced into the vicinity of 5' terminal of a lipase gene pFL-2 containing a lipase gene originated from Pseudomonas fragi IFO 12049 strain, constructed according to the method of the present inventors, disclosed in Japanese patent application No. Sho 63-187,684, according to a site-specific mutagenesis method [Morinaga, Y., Frances-chini, T., Inouye, S. and Inouye, M., Biotechnology, 2, 636 (1984)].

Namely, using the oligonucleotide of 5'-CGTCCATGG* TCAATCC-3' (*: variant-introduced site) 16 mer complementary to the DNA sequence of a lipase initiation codon of the plasmid pFL-2, a restriction enzyme NcoI site was introduced into just the front of the lipase initiation codon.

The resulting plasmid was digested with restriction enzymes NcoI·ScaI, followed by separating and recovering 1Kb DNA fragment containing the lipase gene according to electrophoresis method and further subjecting it to Klenow fragment treatment to thereby make its terminal blunt.

Separately, the plasmid pTC1 constructed according to the method described in the above Example 1 was digested with a restriction enzyme Eco RI, followed by subjecting the digested pTC1 to Klenow fragment treatment to thereby make its terminal blunt. The thus obtained DNA fragment originated from pTC1 and made blunt at its terminal and the previously obtained 1kb DNA fragment containing a lipase gene were reacted in the presence of T4 DNA ligase to obtain a plasmid pTCE-lip as constructed in Fig. 2.

Here, whether the aimed plasmid was obtained or not, was confirmed by the reaction with T4DNA ligase into Escherichia coli, followed by culturing the resulting material on an LB medium containing Ampicillin, preparing a plasmid from an Ampicillin-resistant colony which appeared there and preparing a restriction map thereof.

### (2-2) Construction of plasmid pTCS-lip

The plasmid having the restriction enzyme NcoI site introduced into just the front of the lipase gene initiation codon, obtained above in the item (2-1), was digested with restriction enzymes NcoI.EcoRI, followed by separating and recovering 1.1 Kb DNA fragment containing a lipase gene according to an electrophoresis method.

This 1.1 Kb DNA fragment and 3.1 Kb DNA fragment obtained by digesting a plasmid pTV118N (made by Takara Shuzo Co., Ltd.) with restriction enzymes NcoI·EcoRI were reacted in the presence of T4 DNA ligase to obtain a plasmid pNE118 lip as constructed in Fig. 2.

This plasmid pNE 118 lip was digested with restriction enzyme NcoI, followed by Klenow fragment treatment to make its terminal blunt, digesting with restriction enzyme Hind III and separating and recovering 1.2 Kb DNA fragment containing a lipase gene according to electrophoresis method.

This 1.2 Kb DNA fragment and 4.4 Kb DNA fragment obtained by digesting the plasmid pIC1 obtained above in the item (1) with restriction enzymes SmaI, Hind III were reacted in the presence of T4DNA ligase to obtain a plasmid pTCS-lip as constructed in Fig. 2.

Here, whether the aimed plasmid was obtained or not was confirmed by introducing the reaction product obtained with T4 DNA ligase into Escherichia coli, followed by culturing the resulting material on an LB medium containing Ampicillin, preparing a plasmid from an Ampicillin-resistant colony which appeared there and preparing a restriction map thereof.

### (3) DNA sequence between a lipase gene initiation codon ATG of plasmids pTCE-lip, pTCS-lip and SD sequence originated from tac promoter:

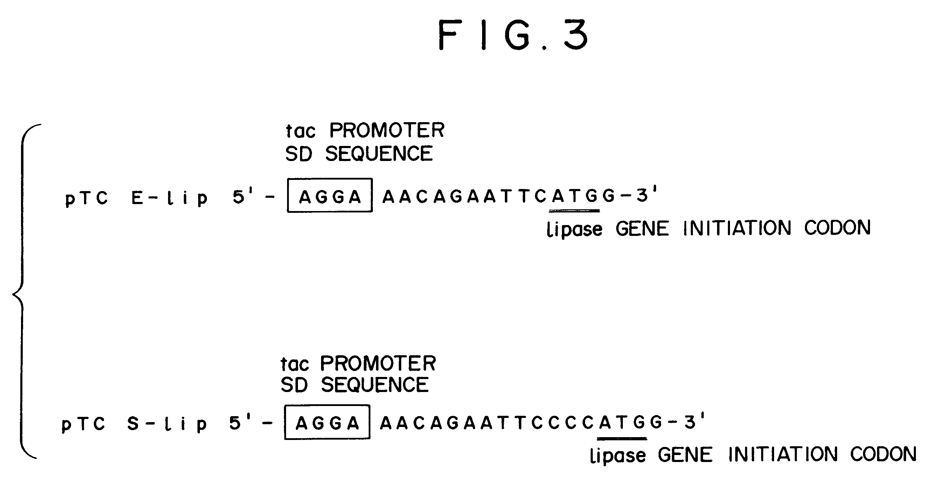

In order to confirm the DNA sequence between the lipase gene initiation codon ATG of pTCE-lip and pTCS-lip obtained above in the item (2) and SD sequence originated from tac promoter, the nucleotide sequence was determined using M13 sequencing kits (made by Takara Shuzo Co., Ltd.), according to Hattori et al's method [M. Hattori and Y. Sasaki, Anal. Biochem., 152, 232 (1986). The results are shown in Fig 3.

### Example 2 (Expression of lipase in Escherichia coli)

The lipase expression plasmids pTCE-lip and pTCS-lip obtained in Example 1 were each introduced into Escherichia coli JM109 (lac I^{q}-generating bacterium) [Yanisch-Perron, C. Vieira, J. and Messing, J., Gene, 33, 103 (1985)], followed by subjecting the resulting substance to shaking culture on an LB medium containing Ampicillin (50 »g/mℓ) at 37°C overnight, inoculating the resulting cultured solution (100 »ℓ) newly on 5mℓ of LB medium containing Ampicillin (50 »g/mℓ), again subjecting the resulting substance to shaking culture at 37°C, adding IPTG 2 hours after the inoculation so as to give a final concentration of 0.6 mM, further continuing shaking culture at 37°C, subjecting 1 mℓ of the cultured solution 3 hours after the culture to centrifugal separation and collecting the resulting bacterial cells of the Escherichia coli.

To the bacterial cells was added 500 »ℓ of 20 mM Tris-HCℓ buffer solution (pH: 8.0), followed by suspending them, and breaking them by means of an ultrasonic wave generator (SONIFIRE 250 type® made by BRANSON Co., Ltd.).

With a portion of the resulting bacterial cells-broken sample was mixed an equal quantity of a sample buffer solution (10% glycerol, 5% 2-mercaptoethanol and 2.3% SDS. 0.0625M Tris-HCℓ, pH 6.8), followed by boiling the mixture for 5 minutes, subjecting the resulting substance to SDS-15% polyacrylamide gel electrophoresis according to Laemmli's method [U.K. Laemmli, Nature, 227, 680 (1970)], and thereafter dyeing the polypeptide in the gel with Coomassie Blue solution.

At that time, JM 109 (pTC1) strain and JM 109 (pTCE-lip) strain and JM 109 (pTCS-lip) strain not subjected to inducing production with IPTG were alike prepared as control samples, followed by electrophoresis. From comparison of electrophoretic patterns with those of the control samples, confirmation of expression of polypeptides originated from lipase genes induced with IPTG in JM 109 (pTCE-lip) strain and JM 109 (pTCS-lip) strain was carried out.

### Example 3 (Evaluation of lipase activity)

Lipase activity was evaluated using a lipase kit S as a commercially available product made by Dainippon Seiyaku Co., Ltd. Thus evaluation was carried out in principle as follows:
Dimethylcapryl tributyrate is hydrolyzed by a lipase to form dimethylcaprol, which reacts quantitatively with 5,5'-dithio-bis(2-nitrobenzoic acid) to form yellow 5-thio-2-nitrobenzoic acid.

Namely, a color-developing reagent (1 mℓ), a sample (50 »ℓ) and an esterase inhibitor (20 »ℓ) were placed in test tubes, followed by mixing them together, placing the respective tubes in a constant temperature bath, allowing them to stand at 30°C for 5 minutes, adding a substrate solution (100 »ℓ), just thereafter incubating at 30°C for 30 minutes under light-shielding, thereafter adding a reaction-terminating solution (2.0 mℓ), mixing them, placing the mixture under light-shielding, and measuring an absorbance of 412 nm within one hour using purified water as a control. The case where a difference between the absorbance of the resulting substance and that of the blank was 0.001 is defined as 1 BALB unit.

As the measurement sample, there was used a supernatant (12,000 r.p.m. × 15 min., using a device HIMAC CR 15B® made by Hitachi Co., Ltd.) obtained by sonication of cells obtained by inducing IPTG (0.6 mM) obtained according to the process of Example 2, followed by culturing the resulting substance for 3 hours.

The respective lipase activities of JM 109 (pTCE-lip) strain, JM 109 (pTCS-lip) strain (each of the present invention) and JM 109 (pFL-2) strain (disclosed in Japanese patent application No. Sho 63-187684) were 220 BALB units, 300 BALB units and 130 BALB units, each per mℓ of the culture solution.

In the above Examples, the plasmid using a restriction enzyme, T4 DNA ligase and Klenow fragment, treatment of DNA fragment and preparation of plasmid from bacterial cells were prepared according to conventional method unless otherwise indicated. As the restriction enzymes, T4 DNA ligase made by Nippon Gene Co., Ltd. was used and as the Klenow fragment, that made by Takara Shuzo Co., Ltd. was used.

## Claims

1. A recombinant plasmid for lipase expression comprising
(a) a vector of pUC9 replicable in Escherichia coli,
(b) a tac promoter,
(c) a lipase gene of Pseudomonas fragi IFO 12049 strain having the following sequence under the rule of said fused promoter: 
10 20 30 40 50 60
ATGGACGATT CGGTAAATAC ACGCTATCCG ATCTTATTGG TACACGGCCT TTTTGGATTC

70 80 90 100 110 120
GACCGGATAG GCTCGCATCA CTACTTTCAT GGTATCAAGC AAGCACTCAA TGAGTGCGGT

130 140 150 160 170 180
GCCAGCGTCT TCGTTCCAAT CATTTCAGCC GCCAACGACA ATGAAGCCCG AGGCGATCAA

190 200 210 220 230 240
CTGCTCAAGC AGATCCACAA CCTGCGCAGG CAGGTCGGTG CACAGCGTGT CAACCTGATC

250 260 270 280 290 300
GGCCACAGCC AGGGCGCCCT TACCGCGCGT TATGTGGCGG CCATCGCCCC TGAACTGATC

310 320 330 340 350 360
GCCTCGGTGA CGTCAGTCAG TGGCCCCAAC CACGGCTCCG AGCTGGCCGA TCGCTTGCGC

370 380 390 400 410 420
CTGGCCTTTG TCCCGGGGAG GCTTGGCGAA ACGGTGGCTG CCGCCCTGAC CACCTCGTTC

430 440 450 460 470 480
AGCGCATTTT TATCCGCCCT CAGCGGCCAC CCGCGCCTGC CCCAAAATGC CTTGAATGCG
 
490 500 510 520 530 540
CTTAACGCCC TGACCACCGA TGGGGTTGCC GCCTTCAACC GCCAATATCC CCAGGGGCTG

550 560 570 580 590 600
CCCGACAGAT GGGGCGGCAT GGGCCCCGCA CAGGTGAATG CTGTGCACTA TTATTCCTGG

610 620 630 640 650 660
AGCGGCATCA TCAAAGGCTC GCGGCTGGCC GAGTCACTCA ACCTGCTCGA CCCCTTGCAC

670 680 690 700 710 720
AATGCCTTGC GTGTGTTTGA CAGCTTTTTT ACCCGCGAAA CCCGAGAAAA CGACGGCATG

730 740 750 760 770 780
GTTGGCCGCT TCAGCTCGCA TCTGGGCCAG GTCATCCGCT CCGACTACCC TCTGGACCAC

790 800 810 820 830 840
CTCGACACCA TCAACCATAT GGCAAGAGGA AGCGCAGGCG CATCAACCCG GTAG
 and
(d) a sequence between SD sequence AGGA and initiation ATG of lipase gene being AACAGAATTC and AACAGAATTCCCC.

2. An Escherichia coli strain transformed by a recombinant plasmid as set forth in claim 1.

3. A process for producing a lipase, which comprises using an Escherichia coli strain as set forth in claim 2.

## Patentansprüche

1. Rekombinantes Plasmid zur Expression von Lipase, umfassend
(a) einen pUC9-Vektor, der in Escherichia coli replizieren kann,
(b) einen tac-Promotor,
(c) ein Lipasegen des Stamms Pseudomonas fragi IFO 12049, das die folgende Sequenz aufweist, die unter der Kontrolle des fusionierten Promotors liegt: 
10 20 30 40 50 60
ATGGACGATT CGGTAAATAC ACGCTATCCG ATCTTATTGG TACACGGCCT TTTTGGATTC

70 80 90 100 110 120
GACCGGATAG GCTCGCATCA CTACTTTCAT GGTATCAAGC AAGCACTCAA TGAGTGCGGT

130 140 150 160 170 180
GCCAGCGTCT TCGTTCCAAT CATTTCAGCC GCCAACGACA ATGAAGCCCG AGGCGATCAA

190 200 210 220 230 240
CTGCTCAAGC AGATCCACAA CCTGCGCAGG CAGGTCGGTG CACAGCGTGT CAACCTGATC

250 260 270 280 290 300
GGCCACAGCC AGGGCGCCCT TACCGCGCGT TATGTGGCGG CCATCGCCCC TGAACTGATC

310 320 330 340 350 360
GCCTCGGTGA CGTCAGTCAG TGGCCCCAAC CACGGCTCCG AGCTGGCCGA TCGCTTGCGC

370 380 390 400 410 420
CTGGCCTTTG TCCCGGGGAG GCTTGGCGAA ACGGTGGCTG CCGCCCTGAC CACCTCGTTC

430 440 450 460 470 480
AGCGCATTTT TATCCGCCCT CAGCGGCCAC CCGCGCCTGC CCCAAAATGC CTTGAATGCG
 
490 500 510 520 530 540
CTTAACGCCC TGACCACCGA TGGGGTTGCC GCCTTCAACC GCCAATATCC CCAGGGGCTG

550 560 570 580 590 600
CCCGACAGAT GGGGCGGCAT GGGCCCCGCA CAGGTGAATG CTGTGCACTA TTATTCCTGG

610 620 630 640 650 660
AGCGGCATCA TCAAAGGCTC GCGGCTGGCC GAGTCACTCA ACCTGCTCGA CCCCTTGCAC

670 680 690 700 710 720
AATGCCTTGC GTGTGTTTGA CAGCTTTTTT ACCCGCGAAA CCCGAGAAAA CGACGGCATG

730 740 750 760 770 780
GTTGGCCGCT TCAGCTCGCA TCTGGGCCAG GTCATCCGCT CCGACTACCC TCTGGACCAC

790 800 810 820 830 840
CTCGACACCA TCAACCATAT GGCAAGAGGA AGCGCAGGCG CATCAACCCG GTAG
 und
(d) eine Sequenz zwischen der SD-Sequenz AGGA und dem Startcodon ATG des Lipasegens, welche AACAGAATTC und AACAGAATTCCCC ist.

2. Escherichia coli Stamm, welcher mit einem rekombinanten Plasmid nach Anspruch 1 transformiert ist.

3. Verfahren zur Herstellung von Lipase, welches die Verwendung eines Escherichia coli Stamms nach Anspruch 2 umfaßt.

## Revendications

1. Plasmide recombinant pour l'expression d'une lipase comprenant :
(a) un vecteur de pUC9 pouvant être répliqué dans Escherichia coli,
(b) un promoteur *tac*,
(c) un gène de lipase de la souche IFO 12049 de Pseudomonas fragi ayant la séquence suivante sous le contrôle de ce promoteur fusionné : 
10 20 30 40 50 60
ATGGACGATT CGGTAAATAC ACGCTATCCG ATCTTATTGG TACACGGCCT TTTTGGATTC

70 80 90 100 110 120
GACCGGATAG GCTCGCATCA CTACTTTCAT GGTATCAAGC AAGCACTCAA TGAGTGCGGT

130 140 150 160 170 180
GCCAGCGTCT TCGTTCCAAT CATTTCAGCC GCCAACGACA ATGAAGCCCG AGGCGATCAA

190 200 210 220 230 240
CTGCTCAAGC AGATCCACAA CCTGCGCAGG CAGGTCGGTG CACAGCGTGT CAACCTGATC

250 260 270 280 290 300
GGCCACAGCC AGGGCGCCCT TACCGCGCGT TATGTGGCGG CCATCGCCCC TGAACTGATC

310 320 330 340 350 360
GCCTCGGTGA CGTCAGTCAG TGGCCCCAAC CACGGCTCCG AGCTGGCCGA TCGCTTGCGC

370 380 390 400 410 420
CTGGCCTTTG TCCCGGGGAG GCTTGGCGAA ACGGTGGCTG CCGCCCTGAC CACCTCGTTC

430 440 450 460 470 480
AGCGCATTTT TATCCGCCCT CAGCGGCCAC CCGCGCCTGC CCCAAAATGC CTTGAATGCG

490 500 510 520 530 540
CTTAACGCCC TGACCACCGA TGGGGTTGCC GCCTTCAACC GCCAATATCC CCAGGGGCTG

550 560 570 580 590 600
CCCGACAGAT GGGGCGGCAT GGGCCCCGCA CAGGTGAATG CTGTGCACTA TTATTCCTGG

610 620 630 640 650 660
AGCGGCATCA TCAAAGGCTC GCGGCTGGCC GAGTCACTCA ACCTGCTCGA CCCCTTGCAC

670 680 690 700 710 720
AATGCCTTGC GTGTGTTTGA CAGCTTTTTT ACCCGCGAAA CCCGAGAAAA CGACGGCATG
 
730 740 750 760 770 780
GTTGGCCGCT TCAGCTCGCA TCTGGGCCAG GTCATCCGCT CCGACTACCC TCTGGACCAC

790 800 810 820 830 840
CTCGACACCA TCAACCATAT GGCAAGAGGA AGCGCAGGCG CATCAACCCG GTAG
 et
(d) une séquence située entre la séquence SD AGGA et le codon ATG d'initiation du gène de lipase étant AACAGAATTC et AACAGAATTCCCC.

2. Souche d'Escherichia coli transformée par un plasmide recombinant suivant la revendication 1.

3. Procédé pour la production d'une lipase, qui comprend l'utilisation d'une souche d'Escherichia coli suivant la revendication 2.
